# EUROPÄISCHE PATENTSCHRIFT

(11) **EP 1 175 237 B1**
(45) Veröffentlichungstag und Bekanntmachung des Hinweises auf die Patenterteilung: **24.09.2003**
(21) Anmeldenummer: 00941904.5
(22) Anmeldetag: 28.04.2000
(51) Int. Cl.: A61L 31/08

(54) **STENT ZUR OFFENHALTUNG GANGARTIGER STRUKTUREN**
STENT FOR HOLDING PASSAGEWAYS OPEN
TUTEUR POUR MAINTENIR OUVERTES DES STRUCTURES DE TYPE CONDUIT

(30) Priorität: 30.04.1999 DE 19921088
(43) Veröffentlichungstag der Anmeldung: 30.01.2002
(73) Patentinhaber: Magforce Applications GmbH, 14050 Berlin (DE)
(72) Erfinder: JORDAN, Andreas, D-14167 Berlin (DE)
(74) Vertreter: Wablat, Wolfgang, Dr.Dr.
(86) Internationale Anmeldenummer: DE0001415
(87) Internationale Veröffentlichungsnummer: WO00066192

(56) Entgegenhaltungen:
- DE-A- 19 726 282
- US-A- 5 178 618
- US-A- 5 197 978
- US-A- 5 571 166
- US-A- 5 840 009

## Beschreibung

Die Erfindung betrifft einen Stent zur Offenhaltung von gangartigen Strukturen im menschlichen Körper und zur Vermeidung von Restenosierungsprozessen durch Wärmeeinwirkung auf diesen.

Bei der Behandlung von stenosierenden Prozessen in gangartigen Hohlorganen, z. B. Gefäßen, Harnwegen und dergleichen, oder von Gefäßaneurismen werden zur Offenhaltung der verengten Strukturen Stents oder tubulare Endoprothesen, d. h. im wesentlichen rohrartige Stützkörper aus Metall und/oder einem Polymermaterial in das betreffende Hohlorgan implantiert. Das Problem bei der Verwendung derartiger Implantate besteht jedoch darin, daß oftmals schon bald nach der Implantation eine Restenose oder Obstruktion auftritt, so daß ein mit erheblichen Risiken verbundener und zudem kostenaufwendiger erneuter Eingriff notwendig ist. Im Falle einer Restenose kardiovaskulärer Stents besteht nicht selten die Notwendigkeit einer aufwendigen Bypass-Operation.

Üblicherweise werden zur angiographisch kontrollierten mechanischen Freilegung des obstruktiven Bereiches des Stents spezielle Katheter in Verbindung mit Mikro- oder Laserwerkzeugen verwendet. Eine derartige Regenerierung kann jedoch maximal zweimal erfolgen. Anschließend muß die Stentkonstruktion durch ein neues Implantat ersetzt werden.

Zur Beseitigung der genannten Nachteile wurde bereits die Verwendung von radioaktiven Stents vorgeschlagen (US-Patent 5 840 009), um im Nahfeld der Strahlung eine erneute Einlagerung von Endothelien oder glatten Muskelzellen innerhalb des Stents zu vermeiden. Hierbei bereitet jedoch die genaue Strahlendosierung Schwierigkeiten und zudem herrscht Unklarheit über deren zytotoxische Wirkung.

Zur Vermeidung der Restenose werden des weiteren Stents mit Beschichtungen aus Anti-Adhäsionsmolekülen (DE 197 13 240), Fibrin/Fibrinogen (US 5 660 873), Silikon (US 5 330 500) oder Kohlenstoff (US 5 163 958) oder Stents mit einem Therapeutika-Abgabesystem (US 5 439 446) beschrieben.

Bekannt sind weiterhin aus thermisch reversibel verformbarem Material ausgebildete Stents (US 5 197 978), die in einen verengten Bereich eines Hohlorgans eingebracht und unter Wärmeeinwirkung mit Hilfe eines Ballonkatheters geweitet werden und wieder in ihre ursprüngliche Form zurückgeführt werden können, die unmittelbar mit einem elektrischen Heizelemente verbunden sind. Schließlich werden in der Literatur (US 5 178 618) auch mittels externer Radiofrequenzwellen auf Temperaturen zwischen 50 und 100°C aufheizbare, expandierbare Stents zur Kanalisierung und Stenosierung von gangartigen Strukturen im menschlichen Körper beschrieben. Die Erzeugung von Wärme in dem Stentmaterial verhindert dabei die Proliferation der glatten Muskelzellen, von denen angenommen wird, daß diese für die Restenose des Stents und die damit verbundenen - eingangs beschriebenen - negativen Folgen verantwortlich sind.

Die Regenerierung von elektrisch leitfähigen, eisenhaltigen restenosierten Stents im Körper ist jedoch insofern nachteilig, als deren durch Hysterese und Wirbelstromverluste bedingte Aufheizung erst bei einer relativ hohen Feldstärke-Frequenz-Kombination möglich ist, bei der es aufgrund der Wirbelstromverluste im elektrisch leitfähigen biologischen Gewebe zu einer Leistungsabsorption an der Oberfläche des Körpers und damit zu einer unerwünschten Überhitzung des peripheren Fettgewebes und anderer unbeteiligter Gewebeteile kommt. Eine Regenerierung sowohl metallischer als auch nichtmetallischer Implantate mit Hilfe von Wärme ist somit bisher nicht möglich.

Der Erfindung liegt daher die Aufgabe zugrunde, einen Stent der eingangs erwähnten Art so auszubilden, daß sowohl bei dessen metallischer als auch nichtmetallischer Ausführung eine beliebige selektive Erwärmung der Prothese einerseits zur Vermeidung einer erneuten Stenosierung oder Obstruktion und andererseits zur Erleichterung des Einwachsen des Stents in dem betreffenden Hohlorgan ermöglicht wird.

Erfindungsgemäß wird die Aufgabe mit einem Stent gelöst, der aus metallischem und/oder nichtmetallischem Material besteht und der einerseits zur Positionsbestimmung durch Magnetresonanztomographie und andererseits zu seiner homogenen und kontrollierten Erwärmung und erhöhten Leistungsaufnahme in einem magnetischen Wechselfeld mit bestimmter, klinisch anwendbarer Feldstärke und Frequenz mit nanoskaligen Teilchen aus einem paramagnetischen Kern und einer an dem Stent haftfähigen Umhüllung beschichtet ist.

Mit dem erfindungsgemäßen Stent-Impiantat, an dessen Oberfläche die nanoskaligen Teilchen in gleichmäßiger Verteilung gebunden sind, ist es sowohl bei metallischer als auch nichtmetallischer Ausführung in einem klinisch anwendbaren Bereich der Feldstärke und der Frequenz des magnetischen Wechselfeldes möglich, eine regelbare, allein auf den Stent beschränkte Temperatur einzustellen, die in einem Bereich geringfügig oberhalb der normalen physiologischen Temperatur ein rasches Einwachsen des Implantats durch Begünstigung der Zellneubildung und in einem Temperaturbereich von 50 bis 60°C eine Regeneration des restenosierten Stents gewährleistet. Die Beschichtung mit den nanoskaligen Teilchen ermöglicht zudem nicht nur bei metallischen, sondern auch bei nichtmetallischen Implantaten eine hohe Leistungsaufnahme bei Feldstärken unterhalb 10 kA/m und in einem klinisch zulässigen Frequenzbereich sowie eine gleichmäßige Erwärmung des Stents. Des weiteren ist aufgrund der vorgesehenen Beschichtung unabhängig von dem verwendeten Implantatwerkstoff eine Positionsbestimmung durch Magnetresonanztomographie möglich.

In weiterer Ausbildung der Erfindung sind die nanoskaligen Teilchen aus einem eisenoxidhaltigen, vorzugsweise aber aus einem aus reinem Eisenoxid bestehenden Kern gebildet, der ferro-, ferri- oder vorzugsweise superparamagnetisch ist und eine Umhüllung aus mindestens einer an dem Kern adsorbierenden Schale aufweist. Die Schale(n) verfügt (verfügen) über zur Bildung von kationischen Gruppen befähigte reaktive Gruppen zur dauerhaften Verbindung der äußeren Schale mit der Oberfläche des Stents. Die Herstellung der nanoskaligen Teilchen erfolgt nach bekannten, beispielsweise in den deutschen Offenlegungsschriften 195 15 820, 196 14 136 und 197 26 282 beschriebenen Methoden.

Aus den Unteransprüchen und der nachfolgenden Beschreibung eines Ausführungsbeispiels ergeben sich weitere Merkmale und zweckmäßige Ausgestaltungen sowie Vorteile der Erfindung.

In einer Versuchsdurchführung wurde der Fibrinogen-Anteil einer Fibrin-Fibrinogen-Lösung mit 15 mg/ml einer Präparation aus mit Aminosilan beschichteten nanoskaligen Teilchen gut vermischt. Anschließend wurde ein als expandierbare Metallkonstruktion ausgebildeter handelsüblicher endovaskulärer Stent in die so vorbereitete Fibrin-Fibrinogen-Lösung getaucht. Bei der darauffolgenden Expansion des so mit den nanoskaligen Teilchen beschichteten Stents mit einem Ballonkatheter blieb die Beschichtung stabil auf der Drahtkonstruktion des Stents erhalten. Der beschichtete Stent wurde in ein mit Wasser gefülltes Röhrchen eingebracht und bei einer Frequenz von 100 kHz einem magnetischen Wechselfeld mit einer Stärke von 10 bis 18 kA/m ausgesetzt. Zum Vergleich wurde auch ein unbeschichteter Stent unter ansonsten gleichen Bedingungen in das Wechselfeld eingebracht. Dabei wurde festgestellt, daß bei dem beschichteten Stent bereits bei einer Feldstärke von 10 kA/m eine ausreichende Leistungsaufnahme und eine entsprechende, zur Regeneration restenosierter Stents erforderliche Erwärmung stattfindet. Hingegen wird der unbeschichtete Stent in diesem klinisch relevanten Feldstärkebereich, in dem keine unzulässige Erwärmung anderer Gewebeteile auftritt, nicht erwärmt. Vielmehr ist eine ausreichend hohe Leistungsadsorption des unbeschichteten Stents und eine damit verbundene Erwärmung sowohl des Stents als auch anderer Gewebeteile erst bei Feldstärken von 15 kA/m und mehr zu verzeichnen. Das heißt, die Leistungsaufnahme des mit den nanoskaligen Teilchen beschichteten Stent bei 10 kA/m entspricht im wesentlichen der des unbeschichteten Stents bei 15 kA/m.

Aus der als Funktion der Feldstärke und der Frequenz berechneten Leistungsaufnahme (W/g) und der Perfusionsgeschwindigkeit in dem Gefäß oder Hohlorgan, in das der Stent implantiert ist, kann der Temperatur-Zeit-Verlauf bei der Erwärmung des Stents im menschlichen Körper durch Anlegen eines magnetischen Wechselfeldes bei einer bestimmten Frequenz berechnet werden. In der praktischen Anwendung wird über einen Stent-Implantationskatheter eine fiber-optische Temperaturmeßsonde unter angiographischer Kontrolle in den Stent eingeführt und die Temperatur während der Einwirkung des magnetischen Wechselfeldes kontrolliert. Zur Beschleunigung des Einwachsens des implantierten Stents wird dieser auf eine Temperatur geringfügig oberhalb des normalen physiologischen Niveaus erwärmt, so daß das Zellwachstum an der Oberfläche des Implantats angeregt wird. Bei einer später erforderlichen Regenerationsbehandlung aufgrund einer Restenosierung des Stent-Implantats wird vor Anlegen des magnetischen Wechselfeldes die Perfusion im Bereich des Stents bestimmt. Die genaue Lage des Implantats kann zuvor durch Kontrastbildung bzw. Abbildung des Stents durch Kernspintomographie exakt festgestellt werden.

## Patentansprüche

1. Stent zur Offenhaltung gangartiger Strukturen und zur Vermeidung von Restenosierungsprozessen durch Wärmeeinwirkung auf diesen, **gekennzeichnet durch** einen rohrartigen Stützkörper aus metallischem und/oder nichtmetallischem Material, der einerseits zur Positionsbestimmung **durch** Magnetresonanztomographie und andererseits zu seiner homogenen und kontrollierten Erwärmung und erhöhten Leistungsaufnahme in einem magnetischen Wechselfeld mit bestimmter, klinisch anwendbarer Feldstärke und Frequenz mit nanoskaligen Teilchen aus einem paramagnetischen Kern mit einer an dem Stent haftenden Umhüllung beschichtet ist.

2. Stent nach Anspruch 1, **dadurch gekennzeichnet, daß** die nanoskaligen Teilchen aus einem eisenoxidhaltigen, ferro-, ferri- oder superparamagnetischen Kern und mindestens einer um diesen gelegten Schale bestehen, die einerseits am Kern adsorbieren kann und andererseits über reaktive, zur Bildung von kationischen Gruppen befähigte Gruppen verfügt und vom Körpergewebe so langsam abgebaut wird, daß eine dauerhafte Bindung der äußeren Schale an die Oberfläche des aus metallischem und/oder nichtmetallischem Material gebildeten Stents besteht.

3. Stent nach Anspruch 2, **dadurch gekennzeichnet, daß** der Kern der nanoskaligen Teilchen aus reinem Eisenoxid besteht und Magnetit und/oder Maghemit umfaßt.

4. Stent nach Anspruch 2, **dadurch gekennzeichnet, daß** der Kern aus reinen Eisenoxidteilchen in Form Fe (II) / Fe (III) im Verhältnis 1:1 bis 1:3 besteht.

5. Stent nach Anspruch 2, **dadurch gekennzeichnet, daß** der Kern aus eisenhaltigen Mischoxiden besteht, wobei der Gehalt an von Eisen verschiedenen Metallatomen nicht größer als 70, vorzugsweise nicht größer als 35 Metallatom-% ist.

6. Stent nach Anspruch 2, **dadurch gekennzeichnet, daß** die reaktiven Gruppen der innersten (kernnächsten) Schale von monomeren Aminosilanen oder Carboxylgruppen gebildet sind.

7. Stent nach einem der Ansprüche 2 bis 6, **dadurch gekennzeichnet, daß** analog der Adsorption der inneren Schale an den Eisenoxidkern deren feste Adsorption an der Stentoberfläche durch Mikroemulsionstechnik, vorzugsweise über durch Tenside vermittelte Reaktionen, erfolgt.

8. Stent nach Anspruch 2, **dadurch gekennzeichnet, daß** eine äußere Schale der nanoskaligen Teilchen zur Ankopplung von Biomolekülen vorgesehen ist.

9. Stent nach Anspruch 8, **dadurch gekennzeichnet, daß** die Biomoleküle fibrinolytisch oder antikoagulant wirkende Enzyme, wie z. B. Proteasen, und/oder Heparin oder Heparin-Derivate sind.

10. Stent nach einem der Ansprüche 1 bis 9, **dadurch gekennzeichnet, daß** der durchschnittliche Durchmesser der nanoskaligen Teilchen kleiner als 100 nm ist, vorzugsweise aber 50 nm und besonders bevorzugt 30 nm nicht überschreitet.

11. Stent nach Anspruch 10, **dadurch gekennzeichnet, daß** die mittlere Teilchengröße zwischen 1 und 40 nm, vorzugsweise zwischen 3 und 30 nm liegt.

12. Stent nach Anspruch 1, **dadurch gekennzeichnet, daß** deren Erwärmung durch ein magnetisches Wechselfeld zur Erzeugung schneller Gradientenfelder im Rahmen der Kernspintomographie vorgesehen ist.

## Claims

1. A stent for keeping open tubular structures and restenosis prevention by heating said stent, **characterized in that** said stent is a tube-shaped support made of a metallic and/or non-metallic material and coated with nanoscale particles that comprise a paramagnetic core and a covering that can adhere to said stent for position detection by MR tomography and for homogeneous and controlled heating and power absorption in an alternating magnetic field with a specific field strength and frequency suitable for clinical use.

2. The stent according to claim 1, **characterized in that** said nanoscale particles consist of a ferromagnetic, ferrimagnetic, or superparamagnetic core and at least one shell surrounding said core that, on the one hand, can adsorb to said core and, on the other hand, comprises reactive groups capable of forming cationic groups and is degraded so slowly by the body tissue that the outer shell durably bonds to the surface of said stent made of metallic and/or non-metallic material.

3. The stent according to claim 2, **characterized in that** said core of nanoscale particles consists of pure iron oxide and includes magnetite and/or maghemite.

4. The stent according to claim 2, **characterized in that** said core consists of pure Fe (II) / Fe (III) iron oxide particles at ratios from 1:1 to 1:3.

5. The stent according to claim 2, **characterized in that** said core consists of iron-containing mixed oxides, the content of non-ferrous metallic atoms being not greater than 70%, preferably not greater than 35% of metallic atoms.

6. The stent according to claim 2, **characterized in that** said reactive groups on the innermost shell (closest to the core) are made up of monomeric aminosilanes or carboxyl groups.

7. The stent according to any one of the preceding claims 2 through 6, **characterized in that** said shell, much as its inner shell adsorbs to said iron oxide core, adsorbs to the stent surface through microemulsion, preferably through tenside-mediated reactions.

8. The stent according to claim 2, **characterized in that** an outer shell of said nanoscale particles is provided for the attachment of biomolecules.

9. The stent according to claim 8, **characterized in that** said biomolecules are fibrinolytic or anti-coagulant enzymes such as proteases and/or heparin or heparin derivatives.

10. The stent according to any one of the preceding claims 1 through 9, **characterized in that** the average diameter of said nanoscale particles is smaller than 100 nm but preferably does not exceed 50 nm and most preferably does not exceed 30 nm.

11. The stent according to claim 10, **characterized in that** the average particle size is between 1 and 40 nm, preferably between 3 and 30 nm.

12. The stent according to claim 10, **characterized in that** it is designed to be heated up by an alternating magnetic field for producing rapid gradient fields within NMR tomography.

## Revendications

1. Stent pour maintenir ouvertes des structures en forme de passage et pour éviter des processus de reformation de sténoses par action de chaleur sur celui-ci, **caractérisé par** un corps de soutien tubulaire en matériau métallique et/ou non métallique qui, d'une part pour déterminer la position par tomographie par résonance magnétique et d'autre part pour son échauffement homogène et contrôlé et pour l'augmentation de la puissance absorbée dans un champ magnétique alternatif avec une intensité de champ et une fréquence déterminées cliniquement applicables, est revêtu d'une enveloppe adhérant au stent et présentant des particules à l'échelle des nanomètres avec un noyau paramagnétique.

2. Stent selon la revendication 1, **caractérisé en ce que** les particules à l'échelle des nanomètres sont constituées d'un noyau contenant de l'oxyde de fer, ferromagnétique, ferri-magnétique ou super-paramagnétique et d'au moins une croûte posée autour de celui-ci qui peut d'une part adsorber sur le noyau et qui dispose d'autre part de groupes réactifs capables de former des groupes cationiques et qui est supprimée par le tissu corporel aussi lentement qu'il résulte une adhésion durable de la croûte extérieure sur la surface du stent formé de matériau métallique et/ou non métallique.

3. Stent selon la revendication 2, **caractérisé en ce que** le noyau des particules à l'échelle des nanomètres est constitué d'oxyde de fer pur ou de magnétite et/ou de maghémite.

4. Stent selon la revendication 2, **caractérisé en ce que** le noyau est constitué de particules d'oxyde de fer pur sous forme de Fe(II) / Fe(III) dans un rapport de 1:1 à 1:3.

5. Stent selon la revendication 2, **caractérisé en ce que** le noyau est constitué d'oxydes mixtes contenant du fer, la teneur en atomes métalliques autres que du fer n'est pas supérieure à 70, de préférence pas supérieure à 35 % en atomes métalliques.

6. Stent selon la revendication 2, **caractérisé en ce que** les groupes réactifs de la croûte la plus interne (la plus proche du noyau) sont formés par des aminosilanes monomères ou par des groupes carboxyliques.

7. Stent selon l'une des revendications 2 à 6, **caractérisé en ce que** par analogie à l'adsorption de la croûte interne au noyau d'oxyde de fer, son adsorption ferme à la surface du stent s'effectue par la technique de micro-émulsion, de préférence via des réactions assistées par des agents tensioactifs.

8. Stent selon la revendication 2, **caractérisé en ce qu'**une croûte extérieure des particules à l'échelle des nanomètres est prévue pour le couplage de bio-molécules.

9. Stent selon la revendication 8, **caractérisé en ce que** les bio-molécules sont des enzymes à effet fibrinolytique ou anticoagulant, comme par exemple des protéases et/ou de l'héparine ou des dérivés d'héparine.

10. Stent selon l'une des revendications 1 à 9, **caractérisé en ce que** le diamètre moyen des particules à l'échelle des nanomètres est inférieur à 100 nm, mais de préférence inférieur à 50 nm et de manière particulièrement préférée inférieur à 30 nm.

11. Stent selon la revendication 10, **caractérisé en ce que** la taille de particules moyenne est comprise entre 1 et 40 nm, de préférence entre 3 et 30 nm.

12. Stent selon la revendication 1, **caractérisé en ce que** son échauffement est assuré par un champ magnétique alternatif pour générer des champs de gradient rapides dans le cadre de la tomographie à spin nucléaire.
